# EUROPEAN PATENT SPECIFICATION

(11) **EP 1 390 045 B1**
(45) Date of publication and mention of the grant of the patent: **03.12.2008**
(21) Application number: 02733745.0
(22) Date of filing: 28.05.2002
(51) Int. Cl.: A61K 31/662, A61P 9/10

(54) **THE USE OF A COMPOUND WITH A NEGATIVELY CHARGED DOMAIN OF PHOSPHOROUS-CONTAINING RADICALS FOR THE TREATMENT OF RESTENOSIS**
VERWENDUNG EINER VERBINDUNG MIT EINER NEGATIV GELADENEN DOMÄNE VON PHOSPHOR-ENTHALTENDEN RADIKALEN FÜR DIE BEHANDLUNG VON RESTENOSIS
UTILISATION DE COMPOSE A DOMAINE A CHARGE NEGATIVE DE RADICAUX PHOSPHOREUX POUR LE TRAITEMENT DE LA RESTENOSE

(30) Priority: 28.05.2001 SE 0101854
(43) Date of publication of application: 25.02.2004
(73) Proprietor: Bioneris AB, c/o Ekonomikonsult Islinge KB, 181 31 Lidingö (SE)
(72) Inventor: SIREN, Matti, Juhani, FI-00170 Helsingfors (FI)
(74) Representative: Larfeldt, Helene
(86) International application number: PCT/SE2002/001016
(87) International publication number: WO 2002/096436

(56) References cited:
- WO-A2-97/24111

## Description

The present invention relates to the use of a compound containing a high density, negatively charged domain of at least three vicinally oriented phosphorous-containing radicals for the preparing of a medicament for preventing, alleviating or combatting restenosis in mammals including man.

Procedures involving the use of balloon dilatation catheters, stents and other percutaneously delivered interventional devices are commonly performed on patients with coronary heart disease (CHD). The balloon angioplasty is the strategy of choice in the majority of cases and the procedure is often followed by stenting. Despite the fact that centres undertaking such procedures are properly equipped and staffed, restenosis, i.e. reocclusion of the vessels, is occurring in 30-50% of the patients treated.

In 1995 nearly 300.000 percutaneous coronary interventions were carried out in Europe and the corresponding figure for the US was approximately 500.000 and these figures continue to increase world wide. Another 500.000 coronary artery bypass procedures were performed in the US. The average cost is more than 20.000 USD per procedure. The costs for the health care system to repeat angioplasty procedures to treat restenosis is estimated to exceed USD 2,5 billion annually.

Restenosis is considered to be one of the major limitations of percutanous transluminal coronary angioplasty. Risk factors for the development of restenosis are multifactoral and mostly unknown. The traditional atherosclerotic risk factors such as hypertension, smoking and cholesterol have not been associated with restenosis. The mechanism for the process of restenosis involves many factors including vasoconstriction, migration and proliferation of smooth muscle cells, the release of regulatory substances such as.growth factors, synthesis of extracellular matrices, neointimal formation, and remodelling of vessels.

In an effort to halt restenosis, more than 50 drugs have been investigated but none have shown to be efficient. As a commonly used compound, heparin is administered during the procedures in order to counteract complications related to increased coagulation but evidence are accumulating towards the direction that heparin could promote the process of restenosis.

According to the present invention it has surprisingly become possible to use a compound containing a high density, negatively charged domain of at least three vicinally oriented phosphorous-containing radicals for the preparing of a medicament for preventing, alleviating or combatting restenosis in mammals including man. Furthermore the invention describes the use of a compound containing a high density, negatively charged domain of at least three vicinally oriented phosphorous-containing radicals for the preparing of a medicament for preventing, alleviating or combatting hyperplasia, remodelling and hypertrophy in mammals including man.

The invention also relates to the use of a compound wherein the phosphorus-containing radicals have the following formula:
a) 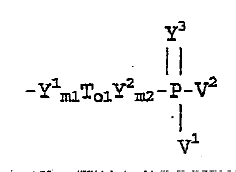 or
b) 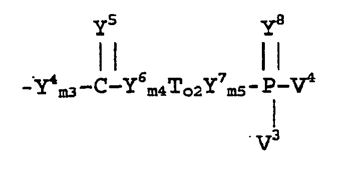
wherein
V¹ to V⁴ are Y⁸ ₘ₆Tₒ₃U
Tₒ₁ to Tₒ₃ are (CH₂)ₙ, CHCH, or CH₂CHCHCH₂
o1 to o3 are 0 to 1
n is 0 to 4
U is R¹Y⁹ ₘ₇, CY¹⁰Y¹¹R², SY¹²Y¹³Y¹⁴R³, PY¹⁵Y¹⁶Y¹⁷R⁴R⁵,
Y¹⁸PY¹⁹Y²⁰Y²¹R⁶R⁷, CH₂NO₂, NHSO₂R⁸ or NHCY²²Y²³R⁹
m1 to m7 are 0 to 1
Y¹ to Y²³ are N R¹⁰, NOR¹¹, O or S
and where R¹ to R¹¹ are
i) hydrogen
ii) a straight or branched saturated or unsaturated alkyl residue containing 1-22 carbon atoms
iii) a saturated or unsaturated aromatic or non-aromatic homo- or heterocyclic residue containing 3-22 carbon atoms and 0-5 heteroatoms consisting of nitrogen, oxygen or sulfur
iv) a straight or branched saturated or unsaturated alkyl residue containing 1-22 carbon atoms substituted with a saturated or unsaturated aromatic or non-aromatic homo- or heterocyclic residue containing 3-22 carbon atoms and 0-5 heteroatoms consisting of nitrogen, oxygen or sulfur
v) an aromatic or non-aromatic homo- or heterocyclic residue containing 3-22 carbon atoms and 0-5 heteroatoms consisting of nitrogen, oxygen or sulfur substituted with a straight or branched saturated or unsaturated alkyl residue containing 1-22 carbon atoms.
   in the said groups ii-v the residues and/or the substituents thereof being substituted with 0-6 of the following groups: hydroxy, alkoxy, aryloxy, acyloxy, carboxy, alkoxycarbonyl, alkoxycarbonyloxy, aryloxycarbonyl, aryloxycarbonyloxy, carbamoyl, fluoro, chloro, bromo, azido, cyano, oxo, oxa, amino, imino, alkylamino, arylamino, acylamino, arylazo, nitro, alkylthio or alkylsulfonyl.

The straight or branched saturated or unsaturated alkyl residue in groups i-v above can be exemplified by methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl, doeicosyl, isopropyl, isobutyl, isopentyl, isohexyl, isoheptyl, isooctyl, isononyl, isodecyl, isodoecosyl, 2-butyl, 2-pentyl, 2-hexyl, 2-heptyl, 2-octyl, 2-nonyl, 2-decyl, 2-doeicosyl, 2- methylbutyl, 2-methylpentyl, 2-methylhexyl, 2-methylheptyl, 2-methyloctyl, 2-methylnonyl, 2- methyldecyl, 2-methyleicosyl, 2-ethylbutyl, 2-ethylpentyl, 2-ethylhexyl, 2-ethylheptyl, 2-ethyloctyl, 2-ethylnonyl, 2-ethyldecyl, 2-ethyleicosyl, tertbutyl, ethenyl, propenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, octadecenyl, nonadecenyl, eicosenyl, heneicosenyl, doeicosenyl, butadienyl, pentadienyl, hexadienyl, heptadienyl, octadienyl, nonadienyl, decadienyl, doeicodienyl, ethynyl, propynyl, doeicosynyl.

The saturated or unsaturated aromatic or non-aromatic homo- or heterocyclic residue in groups i-v above can be exemplified by cyclopropyl, cyclobutyl, cyclopentyl, cyclohexyl, cycloheptyl, cyclooctyl, cyclononyl, cyclodecyl, cycloundecyl, cyclododecyl, cycloridecyl, cyclotetradecyl, cyclopentadecyl, cyclohexadecyl, cycloheptadecyl, cyclooctadecyl, cyclononadecyl, cycloeicosyl, cycloheneicosyl, cyclodoeicosyl, adamantyl, cyclopropenyl, cyclobutenyl, cyclopentenyl, cyclohexenyl, cycloheptenyl, cyclooctenyl, cyclononenyl, cyclodecenyl, phenyl, biphenyl, naphthyl, hydroxyphenyl, aminophenyl, mercaptophenyl, fluorophenyl, chlorophenyl, azidophenyl, cyanophenyl, carboxyphenyl, alkoxyphenyl, acyloxyphenyl, acylphenyl, oxiranyl, thiiranyl, aziridinyl, oxetanyl, thietanyl, azetidinyl, tetrahydrofuranyl, tetrahydrothiophenyl, pyrrolidinyl, tetrahydropyranyl, tetrahydrothiopyranyl, piperidinyl, quinuclidinyl, dioxanyl, dithianyl, trioxanyl, furyl, pyrrolyl, thienyl, pyridyl, quinolyl, benzofuryl, indolyl, benzothienyl, oxazolyl, imidazolyl, thiazolyl, pyridazinyl, pyrimidyl, pyrazinyl, purinyl or a carbohydrate.

Substituents may be selected from the group of:
hydroxy, alkoxy, aryloxy, acyloxy, carboxy, alkoxycarbonyl, aIkoxycarbonyloxy, aryloxycarbonyl, aryloxycarbonyloxy, carbamoyl, fluoro, chloro, bromo, azido, cyano, oxo, oxa, amino, imino, alkylamino, arylamino, acylamino, nitro, alkylthio, alkylsulfonyl.

Furthermore the invention relates to the use wherein the phosphorus-containing radicals have the following formula: 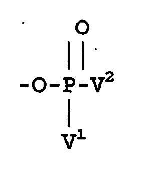 wherein V¹ and V² are OH, (CH₂)ₚ OH, COOH, CONH₂, CONOH, (CH₂)ₚCOOH, (CH₂)ₚCONH₂, (CH₂)ₚCONOH, (CH₂)ₚSO₃ H , (CH₂)ₚSO₃, NH₂, (CH₂)ₚNO₂, (CH₂)ₚPO₃ H₂, O(CH₂)ₚ OH, O(CH₂)ₚ COOH, O(CH₂)ₚCONH₂, O(CH₂)ₚCONOH, (CH₂)ₚSO₃ H, O(CH₂)ₚSO₃ NH₂, O(CH₂)ₚNO₂, O(CH₂)ₚPO₃H₂, CF₂COOH and p is 1 to 4

In this embodiment of the invention the phosphorus-containing radicals are phosphonates or phosphates or derivatives thereof.

In one embodiment of the invention the backbone to the high density negatively charged region of vicinally oriented radicals is a cyclic moiety.

The cyclic moiety comprises a saturated or unsaturated aromatic or non-aromatic homo- or heterocyclic moiety. When the moiety is heterocyclic the heteroatoms are selected from the group of oxygen, nitrogen, sulfur or selenium.

Preferably the cyclic moiety comprises 4 to 24 atoms, most preferably 5 to 18. atoms. The cyclic moiety is for example selected from the group of cyclopentane, cyclohexane, cycloheptane, cyclooctane, inositol, monosacharide, disacharide, trisacharide, tetrasacharide, piperidin, tetrahydrothiopyran, 5-oxotetrahydrothiopyran, 5,5-dioxotetrahydrothiopyran, tetrahydroselenopyran, tetrahydrofuran, pyrrolidine, tetrahydrothiophene, 5-oxotetrahydrothiophene, 5,5-dioxotetrahydrothiophene, tetrahydroselenophene, benzene, cumene, mesitylene, naphtalene and phenantrene. When the cyclic moiety is an inositol it could be selected from the group of alloinositol, cisinositol, epiinositol, D/L-chiroinositol, scylloinositol, myoinositol, mucoinositol and neoinositol.

In one preferred embodiment of the intention the compounds are phosphates, phosphonates or phosphinates of cyclohexane such as 1, 2, 3-β -cyclohexane- 1,2,3-trioltrisphosphate.

In other preferred embodiments of this type of the invention the compounds are phosphates, phosphonates or phosphinates of inositol. The number of phosphate, phosphonate or phosphinate radicals per inositol moiety is at least three. The remaining hydroxyl groups on the inositol moiety may be derivatized in the form of ethers or esters.

In one preferred embodiment the compound is myo-inositol-1,2,6-trisphosphate or myo-inositol- 1,2,3-trisphosphate.

In one most preferred embodiment the compounds are selected from the group of D-myo-inositol-1,2,6-trisphosphate, D-myo-inositol-1,2,6-tris(carboxymetylphosphate), D-myo-inositol-1,2,6-tris(carbomethylphosphonate), D-myo-inositol-1,2,6-tris(hydroxymethylphosphonate),D-3,4,5-tri-O-methyl-myo-inositol-1,2,6-trisphosphate, D-3,4,5-tri-O-hexanoyl-myo-inositol-1,2,6-trisphosphate, D-3,4,5-tri-O-butanoyl-myo-inositol-1,2,6-trisphosphate, D-3,4,5-tri-O-pentanoyl-myo-inositol-1,2,6-trisphosphate, D-3,4,5-tri-O-isobutanoyl-myo-inositol-1,2,6-trisphosphate, D-3,4,5-tri-O-propanoyl-myo-inositol-1,2,6-trisphosphate, D-3,4,5-tri-O-(6-hydroxy-4-oxa)hexanoyl-myo-inositol-1,2,6-trisphosphate, D-3,4,5-tri-O-3-(ethylsulphonyl)propanoyl-myo-inositol-1,2,6-trisphosphate, D-3,4,5-tri-O-3-hydroxypropanoyl-myo-inositol-1,2,6-trisphosphate, D-3,4,5-tri-O-(6-hydroxy)-hexanoyl-myo-inositol-1,2,6-trisphosphate, D-5-O-hexanoyl-myo-inositol-1,2,6-trisphosphate, D-3,4,5-tri-O-phenylcarbamoyl-myo-inositol-1,2,6-trisphosphate, D-3,4,5-tri-O-propanoyl-myo-inositol-1,2,6-tris(carboxymethylphosphate), D-3,4,5-tri-O-butanoyl-myo-inositol-1,2,6-tris(carboxymethylphosphate), D-3,4,5-tri-O-isobutanoyl-myo-inositol-1,2,6-tris(carboxymethylphosphate), D-3,4,5-tri-O-pentanoyl-myo-inositol-1,2,6-tris(carboxymethylphosphate), D-3,4,5-tri-O-hexanoyl-myo-inositol-1,2,6-tris(carboxymethylphosphate), D-3,4,5-tri-O-propanoyl-myo-inositol-1,2,6-tris(carboxymethylpbosphonate), D-3,4,5-tri-o-butanoyl-myo-inositol-1,2,6-tris(carboxymethylphosphonate), D-3,4,5-tri-O-isobutanoyl-myo-inositol-1,2,6-tris(carboxymethylphosphonate), D-3,4,5-tri-O-pentanoyl-myo-inositol-1,2, 6-tris (carboxymethylphospbonate), D-3,4,5-tri-O-hexanoyl-myo-inositol-1,2,6-tris(carboxymethylphosphonate), D-3,4,5-tri-O-propanoyl-myo-inositol-1,2,6-tris(hydroxymethylphosphonate), D-3,4,5-tri-O-butanoyl-myo-inositol-1,2,6-tris(hydroxymethylphosphonate), D-3,4,5-tri-O-isobutanoyl-myo-inositol-1,2,6-tris(hydroxymethylphosphonate), D,-3,4,5-tri-O-pentanoyl-myo-inositol-1,2,6-tris(hydroxymethylphosphonate), D-3,4,5-tri-O-hexmoyl-myo-inositol-1,2,6-tris(hydroxymethyl- .. phosphonate).

When the cyclic moiety is a sacharide it could be selected from the group of D/L-ribose, D/L- arabinose, D/L-xylose, D/L-lyxose, D/L-allose, D/L-altrose, D/L- glucose, D/L-mannose, D/L- gulose, D/L-idose, D/L-galactose, D/L-talose, D/L- ribulose, D/h-xylulose, D/L-psicose, D/L-sorbose, D/L-tagatose and D/L-fructose or derivatives thereof. In preferred embodiments of this type of the invention the compounds are phosphates, phosphonates or phosphinates of sacharides. The number of phosphate, phosphonate or phosphinate radicals per sacharide unit is at least three. The remaining hydroxyl groups on the sacharide moiety may be derivatized in the form of ethers or esters. In many instances the ether form is desired as this type of radical prolongs the stability and half-life in vivo as the susceptibility to enzymatic degradation is reduced.

In one preferred embodiment of this type of the invention the compound is selected from the group of mannose-2,3,4-trisphosphate, galactose-2,3,4-trisphosphate, fructose-2,3,4-trisphosphate, altrose-2,3,4-trisphosphate and rhamnose-2,3,4-trisphosphate. In one most preferred embodiment the compound is selected from the group- of R¹-6-O-R²-α-D-manno-pyranoside-2,3,4-trisphosphate, R¹-6-O-R²-α-D-galacto-pyranoside-2,3,4-trisphosphate, R¹-6-O-R²-α-D-altropyranoside-2,3,4-trisphosphate and R¹-6-O-R²-β-D-fructopyranoside-2,3,4-trisphosphate where R¹ and R² independently are as defined above and preferably are methyl, ethyl, propyl, butyl, pentyl or hexyl. Most preferred compounds in this type of the invention are methyl-6-O-butyl-α-D-mannopyranoside-2,3,4-trisphosphate, methyl-6-O-butyl-α-D-galactopyranoside-2,3,4-trisphosphate, methyl-6-O-butyl-α-D-glycopyranoside-2,3,4-trisphosphate, methyl-6-O-butyl-α-D-altropyranoside-2,3,4-trisphosphate, methyl-6-0-butyl-β-D-fructopyranoside-2,3,4-trisphosphate, 1,5-anhydro-D-arabinitol-2,3,4-trisphosphate, 1,5-anhydroxylitol-2,3,4-trisphosphate, 1,2-O-ethylene-β-D-fructopyranoside-2,3,4-trisphosphate, methyl-α-D-rhamno-pyranoside-2,3,4-trisphosphate, methyl-α-D-mannopyranoside-2,3,4-trisphosphate, methyl-6-O-butyl-α-D-mannopyranoside-2,3,4-tris-(carboxymethylphosphate), methyl-6-O-butyl-α-D mannopyranoside-2,3,4-tris(carboxymethylphosphonate), methyl-6-O-butyl-α-D-mannopyranoside-2,3,4-tris(hydroxymethylphosphonate), methyl-6-O-butyl-α-D-galactopyranoside-2,3,4-tris(carboxymethylphosphate), methyl-6-O-butyl-α-D-galactopyranoside-2,3,4-tris(carboxymethylphosphonate), methyl-6-O-butyl-α-D-galactopyranoside-2,3,4-tris(hydroxymethylphosphonate), methyl-6-O-butyl-α-D-glucopyranoside-2,3,4-tris(carboxymethylphosphate), methyl-6-O-butyl-α-D-glucopyranoside-2,3,4-tris(carboxymethylphosphonate), methyl-6-O-butyl-α-D-glucopyranoside-2,3,4-tris(hydroxymethylphosphonate), methyl-6-O-butyl-α-D-altropyranoside-2,3,4-tris-(carboxymethylphosphate), methyl-6-O-butyl-α-D-altropyranoside-2,3,4-tris-(carboxymethylphosphonate), methyl-6-O-butyl-α-D-altropyranoside-2,3,4-tris-(hydroxymethylphosphonate), methyl-6-O-butyl-β-D-fructopyranoside-2,3,4-tris-(carboxymethylphosphate), methyl-6-O-butyl-β-D-fructopyranoside-2,3,4-tris-(carboxymethylphosphonate), methyl-6-O-butyl-β-D-fructo-pyranoside-2,3,4-tris-(hydroxymethylphosphonate).

In other preferred embodiments of the invention the compounds are phosphates, phosphonates or phosphinates of heterocyclic moieties such as 1,5-dideoxy-1,5-iminoarabinitol-2,3,4-trisphosphate,1,5-dideoxy-1,5-iminoarabinitol-2,3,4-tris-(carboxymethylphosphate), 1,5-dideoxy-1,5-imino-arabinitol-2,3,4-tris(carboxymethylphosphonate), 1,5-dideoxy-1,5-iminoarabinitol-2,3,4-tris(hydroxymethylphosphonate), 1,5-dideoxy-1,5-imino-N-(2-phenylethyl)arabinitol-2,3,4-trisphosphate, 1,5-dideoxy-1,5-imino-N-(2-phenylethyl)-arabinitol-2,3,4-tris(carboxymethylphosphate), 1,5-dideoxy-1,5-imino-N-(2-phenylethyl)arabinitol-2,3,4-tris-(carboxymethylphosphonate), 1,5-dideoxy-1,5-imino-N-(2-phenylethyl) arabinitol-2,3,4-tris (hydroxymethylphosphonate).

Within the process of restenosis, a number of regulatory substances are released. One category of substances are growth factors and the activity of these substances are considered to promote neointimal formation and hyperplasia. Ligand-induced dimerisation is a key event in transmembrane signalling by receptors with tyrosinase kinase activity. Unlike other growth factors such as platelet derived growth factor (PDGF) which are dimeric, fibroblast growth factors (FGF) are monomeric and are unable by themselves to induce activation of FGF receptors. Accordingly, FGF function in concert with for example heparin which induce dimerization and subsequent activation which leads to for example induction of transcriptional factors like early growth response factor 1 (Egr-1). It has been hypothesised that Egr 1 may play a key regulatory role by linking injurious stimuli to the induction of genes directing the expression of effector molecules in endothelial cells, smooth muscle cells, fibroblasts and leukocytes.

Such a complex between fibroblast growth factor 2 (FGF2) and its naturally occurring receptor 1 (FGFR1) has been structurally determined and it can be observed that a positively charged canyon formed by a cluster of basic amino acid residues represent the heparin-binding site. One type of activity of the described compounds according to the invention is to replace or take the place of heparin in this domain and thereby act as a blocking agent of fibroblast growth receptors. The consequence of this activity is to counteract the dimerization and subsequent activation, which leads to a down regulation of the detrimental effects of released substances and thereby a halt in the process of restenosis. Furthermore, another activity of the compounds according to the present invention is to affect the vascular remodelling which is an important factor in the process of restenosis. Remodelling is characterised by thickening and enlargement of existing cells and tissue. The geometry of vessels such as arteries can normally change in response to alterations in the local environment but the process of remodelling counteracts this compensatory response. In response to trauma, tissue such as adventitia, undergo a repair process in which fibroblasts are changed into myofibroblasts. This process is followed by deposition of extracellular matrix and results in geometric remodelling of the tissue. Up-regulation of the expression of the interstitial collagenase or matrix metalloproteinase gene and down-regulation of the synthesis of collagen are other events seen in the process of remodelling and restenosis. When administering the compounds according to the invention, one effect is that the process of remodelling is counteracted evidenced by a diminished transformation of fibroblasts into myofibroblasts, positive effects on collagen synthesis and a reduction of the deposition of extracellular matrix. Furthermore, an improved elasticity of the tissue and vessels are observed which shows a positive effect on the cytoskeletal structure of the vessels. Hypertrophy of the heart occurs in response to cardiac wall stress induced by pressure or volume overload. The result is morphological, molecular and functional changes which is observed as increases in protein content, increase in cell size and so forth. These effects are counteracted by the use of the compounds according to the present invention.

The invention is also intended to be used for the preparing of a medicament for preventing, alleviating or combatting damages related to surgery and transplantation of organs and tissues. In these instances it is beneficial to administer the compounds before, during or after the surgery or transplantation. The effects of the compounds are also to effect wound contraction and to modify the scar formation in relation to for example surgery and transplantation in order to improve the surgical outcome. The administration of the compounds will for example be beneficial in connection to bypass operations.

Furthermore, the administration of the compounds could be in connection to the placement of a stenting material, either by deposition of the compounds to the stenting material or by a local delivery to the injury.

According to the invention the compounds are most often present in a salt form or in a form where only a few of the negative charges are protonated. The salt can contain one or more cations in different combinations. Examples of cations are sodium and potassium ions.

The pharmaceutical composition according to the invention may be administered orally, topically, parentally, rectally or by inhalation spray in dosage forms or formulations comprising conventional, non-toxic pharmaceutically acceptable carriers, adjuvants and vehicles.

The pharmaceutical composition for oral use can be present in different forms such as capsules, granules, tablets, troches, lozenges, aqueous suspensions, dispensable powders, emulsions, syrups or elixirs. When the composition is present in liquid form capsules are preferably utilised. At the use of granules, these preferably have a size of 0,15-2 mm. The granules can either consist of the pharmaceutical composition per se or of the composition and suitable fillers. When the pharmaceutical composition is used in a tablet form, the tablets can have a weight of 50-1500 mg, preferably 50-800 mg and most preferably 100-500 mg.

Formulations for oral use include tablets, which contain the active ingredients in a mixture with non-toxic pharmaceutically acceptable excipients. These excipients may be, for example, inert diluents, such as calcium carbonate, sodium chloride, lactose, calcium phosphate or sodium phosphate; granulating and disintegrating agents, for example, potato starch or alginic acid; binding agents, for example, starch, gelatin or acacia; and lubricating agents, for example, magnesium stearate, stearic acid or talc. The tablets may be uncoated or they may be coated by known techniques to delay desintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

For the parenteral application of the composition of this invention, typical dosage forms include intravenous, intramuscular and intraperitoneal formulations.

For the rectal application of the composition of this intention, typical dosage forms include suppositories, rectal gelatin capsules (solutions and suspensions), and enemas or micro-enemas (solutions and suspensions). Thus, in a typical suppository formulation, any one of the compounds of this invention is combined with any pharmaceutically acceptable suppository base such as cocoa butter, esterified fatty acids, glycerinated gelatin and various water-soluble or dispersible bases like polyethylene glycols and polyoxyethylene sorbitan fatty acid ester. Various additives like salicylates or surfactant materials may be incorporated.

For topical use, creams, ointments, gels, solution or the alike containing the compositions are employed according to methods recognised in the art.

Naturally, the therapeutic dosage range for the compounds of the present intention will vary with the size and needs of the patient and the particular condition or disease symptom being treated.

The administration of the pharmaceutical composition according to the invention can be in a combined dosage form or in separate dosage forms.

For administration to human patients appropriate dosages can routinely be determined by those skilled in this art by extension of the results obtained in animals at various dosages. The preferred dosage for humans falls within the range of 0,1 to 100 mg compound per day and kg body weight, especially 0,1 to 50 mg/day/kg body weight.

According to the present invention a process of preventing, alleviating or combatting restenosis by using a compound containing a high density, negatively charged domain of at least three vicinally oriented phosphorous-containing radicals is described.

The invention also relates to a process wherein a compound containing phosphorus-containing radicals with the following formula is used as described above:
a) 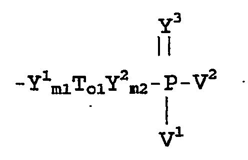 or
b)

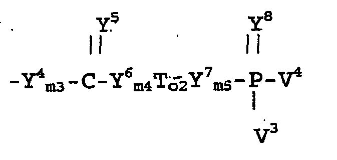

The invention will be further explained with the following embodiment examples however without limiting it thereto.

Example 1 and 2 describe the counteractive effects of D-myo-inositol-1,2,6-trisphosphate (IP₃) and D-3,4,5-tri-O-(phenylcarbamoyl) myo-inositol-1,2,6-trisphosphate (PP 11-201) on the proliferation of smooth muscle cells. Example 3 and 4 show the inhibitory effects of D-myo-inositol-1,2,6-trisphosphate (IP₃) and D-3,4,5-tri-O-(phenylcarbamoyl) myo-inositol-1,2,6-trisphosphate (PP 11- 201) on neointima formation. Example 5 discloses the interaction between the complex of Fibroblast Growth Factor 2 - Fibroblast Growth Factor Receptor 1 (FGF2 - FGFR1) and D-myo-inositol-1,2,6-trisphosphate.

### Example 1

Vascular smooth muscle cell proliferation contributes to restenosis after coronary angioplasty. Human pulmonary artery smooth muscle cells (HPASMCs) were maintained in Medium 231 supplemented with Smooth muscle Growth Supplement. The cells, medium and supplement were obtained from Caascade Biologics Inc., Portland, OR, USA.

The cells were grown as monolayer culture in 100-mm diameter plastic tissue culture dishes (Nunc, Roskilde, Denmark) with 10 ml medium in 37 °C in a humidified atmosphere of 95% air and 5% CO₂ and subcultured at 4- to 6-day intervals. For measurement of cell growth, cells were seeded at density 5,0*10³/well in 96 well dishes in DMEM +0,2% FBS for 3 days. Culture medium was then removed and cells were seeded in DMEM + 0,2% FBS with 1 nM of basic fibroblast growth factor (FGF2). The addition of FGF2 induces cell proliferation of the HPASMCs. The FGF2 used was a recombinant human substance from Boehringer Mannheim Biochemica, Espoo, Finland. Various concentrations of D-myo-inositol-1,2,6-trisphosphate (IP₃) were added and the number of parallel wells in each treatment was four. The proliferation of cells was analysed after 24 hours of incubation by labelling each well with 0,2µCi of ³H-thymidine for two hours. After that the cells were trypsinated and harvested on a filter. A Melt-on scintillator (Meltilex^{™} A; Wallac, Turku, Finland) was allowed to melt on the filter. The radioactivity of the sheets was counted on a liquid scintillator counter (1450 Microbeta Wallac, Turku, Finland).

Addition of FGF2 significantly increased the incorporation of ³H-thymidine in DNA in HPASMCs, which is the measurement of the proliferation of the cells. Stimulation of proliferation was inhibited by the addition of IP₃ with the following result:

| Substance | concentration(µM) | inhibition (%) |
|---|---|---|
| IP₃ | 1 | 61 |
| | 10 | 44 |
| | 100 | 68 |

The experiment shows that there is a strong effect of IP₃ to inhibit stimulated proliferation of smooth muscle cells.

### Example 2

In a procedure similar to the one described in Example 1, D-3,4,5-tri-O-(phenylcarbamoyl) myo- inositol-1,2,6-trisphosphate (PP 11-201) was added with the result shown below:

| Substance | concentration (µM) | inhibition (%) |
|---|---|---|
| PP 11-201 | 0,1 | 49 |
| | 1,0 | 61 |
| | 10 | 70 |

The experiment shows that there is a strong effect of PP 11-201 to inhibit stimulated proliferation of smooth muscle cells.

### Example 3

The effects on neointima formation was assessed after inducing vessel injury in rats with a balloon catheter. Neointima formation is an important part in the process of restenosis. Adult mate Wistar rats (250-260 g) were housed under standard conditions and were fed with commercial rat chow and water ad libitum. During the experiment D-myo-inositol-1,2,6- trisphosphate (IP₃) was administered via subcutaneously operated osmotic minipumps with a dose level of 1 mg/kg/hr. A control group received saline solution by osmotic minipumps. The animals were anaesthetised with an intraperitoneal injection of ketamine, 100 mg/kg, (MTC Pharmaceuticals, Cambridge, Ontario, Canada) and xylazine, 10 mg/kg (Bayer Inc., Etobicoke, Ontario, Canada). Using a dissecting microscope, the left carotid artery was exposed on the ventral side of the neck via a midline incision. The bifurcation of the carotid artery was located and two ligatures were placed around the external carotid artery, which was then tied off with the distal ligature. After temporarily occluding the internal carotid artery with a vascular clamp, a small incision was made between the two ligatures placed around the external carotid artery to introduce the endothelial denudation device, an inflated French Fogarty embolectomy catheter (Baxter Healthcare, Buckinghamshire, UK). Each animal was de-endothelialised by three passages of the catheter and the carotid artery was tied off proximal to the incision hole. The clamp was removed and the pulse of the carotid artery was rechecked. The skin incision was closed with a single suture. 14 days post-operatively, the animals were sedated. An abdominal incision was made to access the abdominal aorta for insertion of a cannula connected to a perfusion apparatus. The animals where killed by an overdose of anaestetic and then perfused with heparinised phosphate buffered saline (pH 7,4) at a rate of 100 ml/min per kg body weight and a pressure of 120 mm Hg. After replacement of saline, 4% paraformaldehyde in isotonic saline was introduced at the same flow rate. After fixation in situ, the carotids were dissected free. Three mid-carotid segments, approximately 10 mm long were rinsed and placed in 4% parafomaldehyde for another 16 hours before embedding and freezing in Tissue- Tek OCT media (Miles Inc., Elkhart, Indiana, USA). Frozen sections were stained with Movat's pentachrome and immunohistochemistry was performed using antibodies in order to examine the process of restenosis by measurement of the neointima formation. It was observed that the administration of IP₃ reduced the formation as can be seen in the following table:

| Substance | Neointima formation (mm²) | Reduction (%) |
|---|---|---|
| Control | 1,12 | |
| IP₃ | 0,48 | 57 |

The experiment shows that the administration of IP₃ reduced neointima formation with 57 %, which describes a beneficial effect against restenosis. By microscopic investigation it could be observed that the elasticity of the vessel walls were improved in the animals receiving IP₃ compared to the vessel walls of the control group which shows a positive effect on remodelling.

### Example 4

In a procedure similar to the one described in Example 3, D-3,4,5-tri-O-(phenylcarbamoyl) myo- inositol-1,2,6-trisphosphate (PP 11-201) was added with the result shown below:

| Substance | Neointima formation (mm²) | Reduction (%) |
|---|---|---|
| Control | 1,12 | |
| PP 11-201 | 0,43 | 62 |

The experiment shows that the administration of PP 11-201 reduced neointima formation with 62 %, which describes a beneficial effect against restenosis. By microscopic investigation it could be observed that the elasticity of the vessel walls were improved in the animals receiving PP 11-201 compared to the vessel walls of the control group which shows a positive effect on remodelling.

### Example 5

The interaction between the complex of Fibroblast Growth Factor 2 - Fibroblast Growth Factor Receptor 1 (FGF2 - FGFR1) and a model compound, D-myo-inositol-1,2,6-trisphosphate, was studied with the Insight modelling package on Silicon Graphics platform using a manual 3-dimensional docking procedure. The protein X-ray structure of FGF2 - FGFR1 was according to Plotnikov et al, Cell 98, 641 (1999), Protein Data Bank entry 1CVS.

A long cleft of the protein complex (30 Å) exposes two binding sites to the model compound with the following characteristics:
Site 1. Near the central part of the cleft there is a concentration of positively charged residues: Lys A26, Lys A135, Lys C163, Lys C166, Lys C172, Lys C175, Lys C207, Arg A120 and. His C166. The following distances between the oxygen in the P-O radical in the model compound and the nitrogen atoms in the positively charged residues were characterized:

| Residue | Distance (Å ) |
|---|---|
| Lys C163 | 2,4 |
| Lys A135 | 2,7 |
| Lys C172 | 2,7 |
| His C166 | 2,3 |

Site 2.
Near the end of the cleft region there is a concentration of another set of positively charged residues: Lys B26, Lys B135, Lys B120, Lys D172, Lys C175, Lys D163, Lys C207, Arg B120 and His D166. The following distances between the oxygen in the P-O radical in the model compound and the nitrogen atoms in the positively charged residues were characterized:

| Residue | Distance (Å ) |
|---|---|
| Lys B135 | 2,3 |
| Lys D163 | 3,6 |
| Lys C175 | 3,2 |
| His D166 | 3,2 |

The modelling experiment shows a strong binding between the model compound, D-myo-inositol-1,2,6-trisphosphate, and two distinct sites of the FGF2 - FGFR1 receptor complex.

## Claims

1. The use of a compound containing a high density negatively charged domain of at least three vicinally oriented phosphorous-containing radicals for the preparing of a medicament for preventing, alleviating or combating restenosis in mammals including man.

2. The use of a compound containing a high density, negatively charged domain of at least three vicinally oriented phosphorous-containing radicals for the preparing of a medicament for preventing, alleviating or combatting hyperplasia, remodelling and hypertrophy in mammals including man.

3. The use according to anyone of claims 1-2 wherein the phosphorus-containing radicals have the following formula:
a) 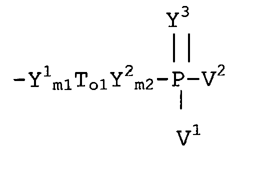 or
b) 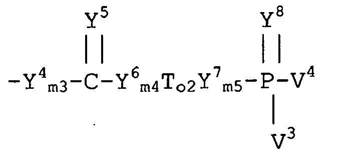
wherein
V¹ to V⁴ are Y⁸ ₘ₆Tₒ₃U
Tₒ₁ to Tₒ₃ are (CH₂)ₙ, CHCH, or CH₂CHCHCH₂
o1 to o3 are 0 to 1
n is 0 to 4
U is R¹Y⁹ₘ₇, CY¹⁰Y¹¹R², SY¹²Y¹³Y¹⁴R³, PY¹⁵Y¹⁶Y¹⁷R⁴R⁵,
Y¹⁸PY¹⁹Y²⁰Y²¹R⁶R⁷, CH₂NO₂, NHSO₂R⁸ or NHCY²²Y²³R⁹
m1 to m7 are 0 to 1
Y¹ to Y²³ are N R¹⁰, NOR¹¹, O or S
and where R¹ to R¹¹ are
i) hydrogen
ii) a straight or branched saturated or unsaturated alkyl residue containing 1-22 carbon atoms
iii)a saturated or unsaturated aromatic or non-aromatic homo- or heterocyclic residue containing 3-22 carbon atoms and 0-5 heteroatoms consisting or nitrogen, oxygen or sulfur
iv) a straight or branched saturated or unsaturated alkyl residue containing 1-22 carbon atoms substituted with a saturated or unsaturated aromatic or non-aromatic homo- or heterocyclic residue containing 3-22 carbon atoms and 0-5 heteroatoms consisting of nitrogen, oxygen or sulfur
v) an aromatic or non-aromatic homo- or heterocyclic residue containing 3-22 carbon atoms and O-5 heteroatoms consisting of nitrogen, oxygen or sulfur substituted with a straight or branched saturated or unsaturated alkyl residue containing 1-22 carbon atoms;
in the said groups ii-v, the residues and/or the substituents thereof being substituted with 0-6 of the following groups: hydroxy, alkoxy, aryloxy, acyloxy, carboxy, alkoxycarbonyl, alkoxycarbonyloxy, aryloxycarbonyl, aryloxycarbonyloxy, carbamoyl, fluoro, chloro, bromo, azido, cyano, oxo, oxa, amino, imino, alkylamino, arylamino, acylamino, arylazo, nitro, alkylthio or alkylsulfonyl.

4. The use according to claim 3 wherein the phosphorus containing radicals have the following formula: 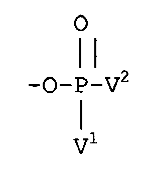 wherein V¹ and V² are OH, (CH₂)ₚ OH, COOH, CONH₂, CONOH, (CH₂)ₚCOOH, (CH₂)ₚCONH₂, (CH₂)ₚCONOH, (CH₂)ₚSO₃H, (CH₂)ₚSO₃ NH₂, (CH₂)ₚNO₂, (CH₂)ₚPO₃H₂, O(CH₂)ₚ OH, O(CH₂)ₚ COOH, O(CH₂)ₚCONH₂, O(CH₂)ₚCONOH, (CH₂)ₚSO₃H, O(CH₂)ₚSO₃NH₂, O(CH₂)ₚNO₂, O(CH₂)ₚPO₃H₂, CF₂COOH and p is 1 to 4

5. The use according to claim 3 wherein the phosphorus-containing radicals are phosphate groups.

6. The use according to anyone of claims 1-2 wherein a backbone to the high density negatively charged region of vicinally oriented radicals is a cyclic moiety.

7. The use according to claim 6 wherein the backbone is a saturated or unsaturated aromatic or non-aromatic homo- or heterocyclic moiety where the heteroatom is nitrogen, oxygen, sulfur or selenium.

8. The use according to claim 7 wherein the cyclic moiety comprises 4 to 24 atoms, preferably 5 to 18 atoms.

9. The use according to claim 7 wherein the cyclic moiety is selected from the group of cyclopentane, cyclohexane, cycloheptane, inositol, monosacharide, disacharide, trisacharide, tetrasacharide, piperidin, tetrahydrothiophyran, 5-oxotetrahydrothiopyran, 5,5-dioxotetrahydrothiopyran, tetrahydroselenophyran, tetrahydrofuran, pyrrolidine, tetrahydrothiophene, 5-oxotetrahydrothiophene, 5,5-dioxotetrahydrothiophene, tetrahydroselenophene, benzene, cumene, mesitylene, naphtalene and phenanthrene.

10. The use according to claim 7 wherein the cyclic moiety is selected from the group of alloinositol, cisinositol, epiinositol, D/L-chiroinositol, scylloinositol, myoinositol, mucoinositol and neoinositol.

11. The use according to claim 7 wherein the cyclic moiety is selected from the group of D/L-ribose, D/L-arabinose, D/L-xylose, D/L-lyxose, D/L-allose, D/L-altrose, D/L-glucose, D/L-mannose, D/L-gulose, D/L-idose, D/L-galactose, D/L-talose, D/L-ribulose, D/L-xylulose, D/L-psicose, D/L-sorbose, D/L-tagatose and D/L-fructose.

12. The use according to claim 7 wherein one of the phosphorus-containing radicals is axial, and two of the phosphorus-containing radicals are equatorial.

13. The use according to claim 12 wherein the compound is selected from the group of myo-inositol-1,2,6-trisphosphate, mannose-2,3,4-trisphosphate, rhamnose-2,3,4-trisphosphate, galactose-2,3,4-trisphosphate, methyl-6-O-butyl-α-D-mannopyranoside-2,3,4- trisphosphate, 1,5-anhydro-D-arabinitol-2,3,4-trisphosphate, fructose-2,3,4-trisphosphate, 1,2-O-ethylene-β-D-fructopyranoside-2,3,4-trisphosphate, cyclohexane-1,2,3-triol trisphosphate, 1,5-dideoxy-1,5-iminoarabinitol-2,3,4-trisphosphate, altrose-2,3,4-trisphosphate, methyl-6-O-butyl-α-D-altropyranoside-2,3,4-trisphosphate or derivatives thereof.

14. The use according to anyone of claims 1-2 wherein the compound is administered by parenteral or non-parenteral administration.

15. The use according to anyone of claims 1-2 wherein the effective amount is from about 0.1 to about 100 mg per kg bodyweight of the animal or man.

16. The use according to anyone of claims 1-2 wherein the medicament is in unit dosage form comprising tablets, granules, capsules, solutions or suspensions.

## Patentansprüche

1. Verwendung einer Verbindung, die eine hochdichte, negativ geladene Domäne von mindestens drei benachbart ausgerichteten, Phosphor enthaltenden Radikalen enthält, zur Herstellung eines Arzneimittels zum Verhindern, Lindern oder Bekämpfen von Restenose in Säugetieren, einschließlich Menschen.

2. Verwendung einer Verbindung, die eine hochdichte, negativ geladene Domäne von mindestens drei benachbart ausgerichteten, Phosphor enthaltenden Radikalen enthält, zur Herstellung eines Arzneimittels zum Verhindern, Lindern oder Bekämpfen von Hyperplasie, Remodelling und Hypertrophie in Säugetieren, einschließlich Menschen.

3. Verwendung nach einem der Ansprüche 1 - 2, wobei die Phosphor enthaltenden Radikale die folgende Formel aufweisen:
a) 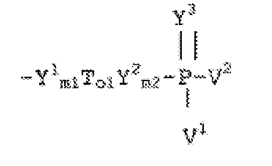 oder
b) 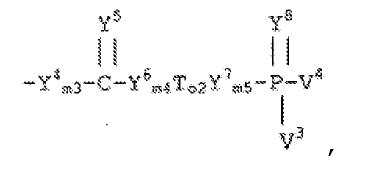
wobei
V¹ bis V⁴ Y⁸ₘ₆Tₒ₃U sind,
Tₒ₁ bis Tₒ₃ (CH₂)ₙ, CHCH oder CH₂CHCHCH₂ sind,
o1 bis o3 0 bis 1 sind,
n 0 bis 4 ist,
U R¹Y⁹ₘ₇, CT¹⁰Y¹¹R², SY¹²Y¹³Y¹⁴R³, PY¹⁵Y¹⁶Y¹⁷R⁴R⁵, Y¹⁸PY¹⁹Y²⁰Y²¹R⁶R⁷,
CH₂NO₂, NHSO₂R⁸ oder NHCY²²Y²³R⁹ ist,
m1 bis m7 0 bis 1 sind,
Y¹ bis Y²³ NR¹⁰, NOR¹¹, O oder S sind,
und wobei R¹ bis R¹¹
i) Wasserstoff,
ii) ein gerader oder verzweigter, gesättigter oder ungesättigter Alkylrest, der 1 - 22 Kohlenstoffatome enthält,
iii) ein gesättigter oder ungesättigter, aromatischer oder nicht aromatischer, homo- oder heterocyclischer Rest, der 3 - 22 Kohlenstoffatome und 0 - 5 Heteroatome, die aus Stickstoff, Sauerstoff oder Schwefel bestehen, enthält,
iv) ein gerader oder verzweigter, gesättigter oder ungesättigter Alkylrest, der 1 - 22 Kohlenstoffatome enthält, substituiert mit einem gesättigten oder ungesättigten, aromatischen oder nicht aromatischen, homo- oder heterocyclischen Rest, der 3 - 22 Kohlenstoffatome und 0 - 5 Heteroatome, die aus Stickstoff, Sauerstoff oder Schwefel bestehen, enthält,
v) ein aromatischer oder nicht aromatischer, homo- oder heterocyclischer Rest, der 3 - 22 Kohlenstoffatome und 0 - 5 Heteroatome, die aus Stickstoff, Sauerstoff oder Schwefel bestehen, enthält, substituiert mit einem geraden oder verzweigten, gesättigten oder ungesättigten Alkylrest, der 1 - 22 Kohlenstoffatome enthält, sind;
wobei in den besagten Gruppen ii - v die Reste und/oder die Substituenten davon mit 0 - 6 Gruppen der folgenden Gruppen substituiert sind: Hydroxy, Alkoxy, Aryloxy, Acyloxy, Carboxy, Alkoxycarbonyl, Alkoxycarbonyloxy, Aryloxycarbonyl, Aryloxycarbonyloxy, Carbamoyl, Fluor, Chlor, Brom, Azido, Cyano, Oxo, Oxa, Amino, Imino, Alkylamino, Arylamino, Acylamino, Arylazo, Nitro, Alkylthio oder Alkylsulfonyl substituiert sind.

4. Verwendung nach Anspruch 3, wobei die Phosphor enthaltenden Radikale die folgende Formel aufweisen: 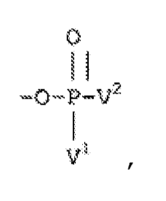 wobei V¹ und V² OH, (CH₂)ₚOH, COOH, CONH₂, CONOH, (CH₂)ₚCOOH, (CH₂)ₚCONH₂, (CH₂)ₚCONOH, (CH₂)ₚSO₃H, (CH₂)ₚSO₃NH₂, (CH₂)ₚNO₂, (CH₂)ₚPO₃H₂, O(CH₂)ₚOH, O(CH₂)ₚCOOH, O(CH₂)ₚCONH₂, O(CH₂)ₚCONOH, (CH₂)ₚSO₃H, O(CH₂)ₚSO₃NH₂, O(CH₂)ₚNO₂, O(CH₂)ₚPO₃H₂, CF₂COOH sind und p 1 bis 4 ist.

5. Verwendung nach Anspruch 3, wobei die Phosphor enthaltenden Radikale Phosphatgruppen sind.

6. Verwendung nach einem der Ansprüche 1 - 2, wobei eine Hauptkette der hochdichten, negativ geladenen Region von benachbart ausgerichteten Radikalen ein cyclischer Teil ist.

7. Verwendung nach Anspruch 6, wobei die Hauptkette ein gesättigter oder ungesättigter, aromatischer oder nicht aromatischer, homo- oder heterocyclischer Teil ist, wobei das Heteroatom Stickstoff, Sauerstoff, Schwefel oder Selen ist.

8. Verwendung nach Anspruch 7, wobei der cyclische Teil 4 bis 24 Atome, vorzugsweise 5 bis 18 Atome umfasst.

9. Verwendung nach Anspruch 7, wobei der cyclische Teil aus der Gruppe aus Cyclopentan, Cyclohexan, Cycloheptan, Inositol, Monosaccharid, Disaccharid, Trisaccharid, Tetrasaccharid, Piperidin, Tetrahydrothiopyran, 5-Oxotetrahydrothiopyran, 5,5-Dioxotetrahydrothiopyran, Tetrahydroselenopyran, Tetrahydrofuran, Pyrrolidin, Tetrahydrothiophen, 5-Oxotetrahydrothiophen, 5,5-Dioxotetrahydrothiophen, Tetrahydroselenophen, Benzol, Cumol, Mesitylen, Naphthalin und Phenanthren ausgewählt ist.

10. Verwendung nach Anspruch 7, wobei der cyclische Teil aus der Gruppe aus Alloinositol, Cisinositol, Epiinositol, D/L-Chiroinositol, Scylloinositol, Myoinositol, Mucoinositol und Neoinositol ausgewählt ist.

11. Verwendung nach Anspruch 7, wobei der cyclische Teil aus der Gruppe aus D/L-Ribose, D/L-Arabinose, D/L-Xylose, D/L-Lyxose, D/L-Allose, D/L-Altrose, D/L-Glucose, D/L-Mannose, D/L-Gulose, D/L-Idose, D/L-Galactose, D/L-Talose, D/L-Ribulose, D/L-Xylulose, D/L-Psicose, D/L-Sorbose, D/L-Tagatose und D/L-Fructose ausgewählt ist.

12. Verwendung nach Anspruch 7, wobei eines der Phosphor enthaltenden Radikale axial ist und zwei der Phosphor enthaltenden Radikale äquatorial sind.

13. Verwendung nach Anspruch 12, wobei die Verbindung aus der Gruppe aus Myoinositol-1,2,6-trisphosphat, Mannose-2, 3,4-trisphosphat, Rhamnose-2,3,4-trisphosphat, Galactose-2,3,4-trisphosphat, Methyl-6-O-butyl-α-D-manno-pyranosid-2,3,4-trisphosphat, 1,5-Anhydro-D-arabinitol-2,3,4-trisphosphat, Fructose-2,3,4-trisphosphat, 1,2-O-Ethylen-α-D-fructopyranosid-2,3,4-trisphosphat, Cyclohexan-1,2,3-trioltrisphosphat, 1,5-Didesoxy-1,5-iminoarabinitol-2,3, 4-trisphosphat, Altrose-2,3,4-tris-phosphat, Methyl-6-0-butyl-α-D-altropyranosid-2,3,4-tris-phosphat oder Derivaten davon ausgewählt ist.

14. Verwendung nach einem der Ansprüche 1 - 2, wobei die Verbindung mittels parenteraler oder nicht-parenteraler Verabreichung verabreicht wird.

15. Verwendung nach einem der Ansprüche 1 - 2, wobei die wirksame Menge von etwa 0,1 bis etwa 100 mg pro kg Körpergewicht des Tiers oder Menschen ausmacht.

16. Verwendung nach einem der Ansprüche 1 - 2, wobei das Arzneimittel in einer Einheitsdosisform ist, die Tabletten, Granulat, Kapseln, Lösungen oder Suspensionen umfasst.

## Revendications

1. Utilisation d'un composé contenant un domaine chargé négativement à haute densité d'au moins trois radicaux contenant du phosphore à orientation vicinale pour la préparation d'un médicament pour prévenir, soulager ou lutter contre la resténose chez des mammifères, y compris l'homme.

2. Utilisation d'un composé contenant un domaine chargé négativement à haute densité d'au moins trois radicaux contenant du phosphore à orientation vicinale pour la préparation d'un médicament pour prévenir, soulager ou lutter contre l'hyperplasie, le remodelage et l'hypertrophie chez des mammifères, y compris l'homme.

3. Utilisation selon l'une quelconque des revendications 1 et 2, dans laquelle les radicaux contenant du phosphore possèdent les formules suivantes :
a) 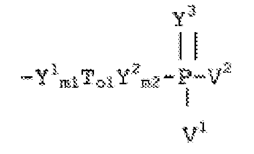 ou
b) 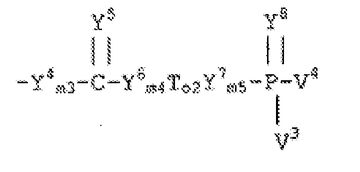
dans lesquelles
V¹ à V⁴ sont Y⁸ₘ₆Tₒ₃U
Tₒ₁ à Tₒ₃ sont (CH₂)ₙ, CHCH ou CH₂CHCHCH₂
o1 à o3 sont 0 à 1
n est 0 à 4
U est R¹Y⁹ₘ₇, CY¹⁰Y¹¹R², SY¹²Y¹³Y¹⁴R³, PY¹⁵Y¹⁶Y¹⁷R⁴R⁵,
Y¹⁸PY¹⁹Y²⁰Y²¹R⁶R⁷, CH₂NO_{2,} NHSO₂R⁸ ou NHCY²²Y²³R⁹
m1 à m7 sont 0 à 1
Y¹ à Y²³ sont NR¹⁰, NOR¹¹, O ou S
et où R¹ à R¹¹ sont
i) un hydrogène
ii) un résidu alkyle, saturé ou insaturé, linéaire ou ramifié, contenant 1 à 22 atomes de carbone
iii) un résidu homo- ou hétérocyclique, aromatique ou non aromatique, saturé ou insaturé, contenant 3 à 22 atomes de carbone et 0 à 5 hétéroatomes consistant en l'azote, l'oxygène ou le soufre
iv) un résidu alkyle, saturé ou insaturé, linéaire ou ramifié, contenant 1 à 22 atomes de carbone substitué avec un résidu homo- ou hétérocyclique, aromatique ou non aromatique, saturé ou insaturé, contenant 3 à 22 atomes de carbone et 0 à 5 hétéroatomes consistant en l'azote, l'oxygène ou le soufre
v) un résidu homo- ou hétérocyclique, aromatique ou non aromatique, contenant 3 à 22 atomes de carbone et 0 à 5 hétéroatomes consistant en l'azote, l'oxygène ou le soufre substitué avec un résidu alkyle, saturé ou insaturé, linéaire ou ramifié, contenant 1 à 22 atomes de carbone ;
dans lesdits groupes ii à v, les résidus et/ou leurs substituants étant substitués avec 0 à 6 des groupes suivants : hydroxy, alcoxy, aryloxy, acyloxy, carboxy, alcoxycarbonyle, alcoxycarbonyloxy, aryloxycarbonyle, aryloxycarbonyloxy, carbamoyle, fluoro, chloro, bromo, azido, cyano, oxo, oxa, amino, imino, alkylamino, arylamino, acylamino, arylazo, nitro, alkylthio ou alkylsulfonyle.

4. Utilisation selon la revendication 3, dans laquelle les radicaux contenant du phosphore possèdent la formule suivante : 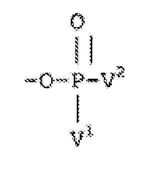 dans laquelle V¹ et V² sont OH, (CH₂)ₚOH, COOH, CNH₂, CONOH, (CH₂)ₚCOOH, (CH₂)ₚCONH₂, (CH₂)ₚCONOH, (CH₂)ₚSO₃H, (CH₂)ₚSO₃NH₂, (CH₂)ₚNO₂, (CH₂)ₚPO₃H₂, O(CH₂)ₚOH, O(CH₂)ₚCOOH, O(CH₂)ₚCONH₂, O(CH₂)ₚCONOH, (CH₂)ₚSO₃H, O(CH₂)ₚSO₃NH₂, O(CH₂)ₚNO₂, O(CH₂)ₚPO₃H₂, CF₂COOH et p est 1 à 4.

5. Utilisation selon la revendication 3, dans laquelle les radicaux contenant du phosphore sont des groupes phosphates.

6. Utilisation selon l'une quelconque des revendications 1 et 2, dans laquelle un squelette de la région chargée négativement à haute densité de radicaux à orientation vicinale est un fragment cyclique.

7. Utilisation selon la revendication 6, dans laquelle le squelette est un fragment homo- ou hétérocyclique, aromatique ou non aromatique, saturé ou insaturé, où l'hétéroatome est l'azote, l'oxygène, le soufre ou le sélénium.

8. Utilisation selon la revendication 7, dans laquelle le fragment cyclique comprend 4 à 24 atomes, de préférence 5 à 18 atomes.

9. Utilisation selon la revendication 7, dans laquelle le fragment cyclique est choisi dans le groupe comprenant le cyclopentane, le cyclohexane, le cycloheptane, l'inositol, un monosaccharide, un disaccharide, un trisaccharide, un tétrasaccharide, la pipéridine, le tétrahydrothiophyrane, le 5-oxotétrahydrothiopyrane, le 5,5-dioxotétrahydro-thiopyrane, le tétrahydrosélénophyrane, le tétrahydro-furane, la pyrrolidine, le tétrahydrothiophène, le 5-oxo-tétrahydrothiophène, le 5,5-dioxotétrahydrothiophène, le tétrahydrosélénophène, le benzène, le cumène, le mésitylène, le naphtalène et le phénanthrène.

10. Utilisation selon la revendication 7, dans laquelle le fragment cyclique est choisi dans le groupe comprenant l'alloinositol, le cisinositol, l'épiinositol, le D/L-chiroinositol, le scylloinositol, le myoinositol, le mucoinositol et le néoinositol.

11. Utilisation selon la revendication 7, dans laquelle le fragment cyclique est choisi dans le groupe comprenant le D/L-ribose, le D/L-arabinose, le D/L-xylose, le D/L-lyxose, le D/L-allose, le D/L-altrose, le D/L-glucose, le D/L-mannose, le D/L-gulose, le D/L-idose, le D/L-galactose, le D/L-talose, le D/L-ribulose, le D/L-xylulose, le D/L-psicose, le D/L-sorbose, le D/L-tagatose et le D/L-fructose.

12. Utilisation selon la revendication 7, dans laquelle un des radicaux contenant du phosphore est axial et deux des radicaux contenant du phosphore sont équatoriaux.

13. Utilisation selon la revendication 12, dans laquelle le composé est choisi dans le groupe comprenant le myo-inositol-1,2,6-trisphosphate, le mannose-2,3,4-trisphosphate, le rhamnose-2,3,4-trisphosphate, le galactose-2,3,4-trisphosphate, le méthyl-6-O-butyl-α-D-manno-pyranoside-2,3,4-trisphosphate, le 1,5-anhydro-D-arabinitol-2,3,4-trisphosphate, le fructose-2,3,4-trisphosphate, le 1,2-O-éthylène-α-D-fructopyranoside-2,3,4-trisphosphate, le cyclohexane-1,2,3-triol trisphosphate, le 1,5-didésoxy-1,5-iminoarabinitol-2,3,4-trisphosphate, l'altrose-2,3,4-trisphosphate,le méthyl-6-O-butyl-α-D-altropyranoside-2,3,4-trisphosphate ou leurs dérivés.

14. Utilisation selon l'une quelconque des revendications 1 et 2, dans laquelle le composé est administré par administration parentérale ou non parentérale.

15. Utilisation selon l'une quelconque des revendications 1 et 2, dans laquelle la quantité efficace est d'environ 0,1 à environ 100 mg par kg de poids corporel de l'animal ou de l'homme.

16. Utilisation selon l'une quelconque des revendications 1 et 2, dans laquelle le médicament est sous une forme galénique unitaire comprenant les comprimés, les granulés, les capsules, les solutions ou les suspensions.
